# FASCICULE DE BREVET EUROPEEN

(11) **EP 1 229 872 B1**
(45) Date de publication et mention de la délivrance du brevet: **31.08.2005**
(21) Numéro de dépôt: 00977637.8
(22) Date de dépôt: 09.11.2000
(51) Int. Cl.: A61F 2/34

(54) **PROTHESE COTYLOIDIENNE EXPANSIBLE A DOUBLE MOBILITE**
DEHNBARE HÜFTPFANNENPROTHESE MIT ZWEI FREIHEITSGRADEN
EXPANSIBLE ACETABULAR PROSTHESIS WITH DOUBLE MOBILITY

(30) Priorité: 19.11.1999 FR 9914543
(43) Date de publication de la demande: 14.08.2002
(73) Titulaire: Proconcept SA, 84350 Courthezon (FR)
(72) Inventeur: AFRIAT, Jacques, F-11100 Narbonne (FR); BENSADOUN, Jean-Louis, F-42600 Lezigneux (FR)
(74) Mandataire: Schmitt, John
(86) Numéro de dépôt international: PCT/FR2000/003115
(87) Numéro de publication internationale: WO 2001/035873

(56) Documents cités:
- EP-A- 0 486 403
- EP-A- 0 640 325
- WO-A-97/38650
- FR-A- 2 645 433
- FR-A- 2 680 674
- FR-A- 2 700 946
- US-A- 4 834 759

## Description

La présente invention a pour objet une prothèse cotyloïdienne expansible à double mobilité.

Le secteur technique de l'invention est le domaine de la réalisation de matériaux chirurgicaux implantables dans le corps humain.

L'application principale de l'invention est la réalisation de prothèse de hanche pour remplacer la cavité iliaque de l'os de la hanche, l'ensemble devant être ancré sans ciment.

Les prothèses totales de la hanche comportent deux éléments :
1. La tige fémorale : cette pièce remplace la tête et le col du fémur. Une tige, prolongement du col, en assure l'ancrage dans l'os fémoral, avec ou sans ciment.
2. La pièce cotyloïdienne : elle remplace le cotyle osseux et s'articule avec la tête fémorale prothétique. Il s'agit pour la surface de glissement d'une cavité hémisphérique. Cet élément peut être composé d'une seule pièce en métal, polyéthylène ou céramique. Toutefois, pour en permettre la fixation sans ciment, de nombreux modèles se composent des deux éléments suivants :
   a) une cupule métallique fixée dans l'os,
   b) un insert en matière plastique, notamment polyéthylène, fixé dans la cupule et s'articulant avec la tête prothétique.

Les problèmes rencontrés dans la pratique chirurgicale sont l'usure entre la tête fémorale et la pièce cotyloïdienne, mais aussi la fixation de la cupule métallique dans l'os et l'usure observée entre l'insert et la cupule métallique.

Plusieurs modes de fixation de la cupule métallique sont actuellement utilisés, notamment le scellement (a) ou l'impaction en force (b), le vissage (c) ou encore la fixation par expansion (d), qui sont commentés ci-après.
a) Le scellement consiste à interposer un ciment de type acrylique entre l'os et la cupule, qui s'ancre dans l'os et assure la fixation de la pièce. Mais cette technique présente des inconvénients liés aux caractéristiques du ciment et de l'os, et peut conduire à moyen ou long terme à un descellement.
b) L'impaction en force consiste à entrer en force ("press-fit") la cupule métallique dans une cavité osseuse préparée à l'aide de fraises hémisphériques, qui calibrent le cotyle osseux à un diamètre légèrement inférieur à celui de la cupule métallique.
   Un revêtement ou des aspérités de surface situées sur la surface extérieure de cette cupule améliorent la tenue mécanique immédiate de la cupule et permettent une tenue secondaire par repousse osseuse. Mais cette tenue mécanique initiale est aléatoire car elle dépend pour beaucoup du mode de préparation de l'os, et surtout de sa dureté.
   La plupart des modèles proposent donc une fixation complémentaire, recommandée dans les cas où la tenue en "press-fit" est jugée insuffisante. Cette fixation est assurée par des vis qui sont mises en place dans l'os à travers des trous aménagés dans la cupule métallique.
   On reproche à cette technique d'impaction en force les inconvénients suivants:
   - la micromobilité possible entre la vis et la cupule métallique, peut libérer des produits d'usure métalliques particulièrement nocifs, et
   - le contact possible entre la tête de vis et l'insert, est source possible d'usure et de fluage. Egalement redouté est le fluage de l'insert (s'il est en polyéthylène) en regard des trous de vis, car il produit des débris d'usure qui peuvent migrer à travers les trous de vissage et aboutir à une ostéolyse progressive, laquelle favorise la mobilisation de l'implant.
c) Le vissage du cotyle consiste à utiliser une cupule métallique présentant un filetage sur sa surface extérieure, lequel filetage est vissé dans l'os. Ce mode de fixation est actuellement peu utilisé car le positionnement de la pièce, lors de la mise en place, est aléatoire. En effet, les cotyles press-fit ou vissés ne peuvent pas être posés avec précision car leur positionnement peut être dévié par l'os lors de la mise en place.
d) Dans la fixation par expansion, on utilise une cupule métallique qui est conçue de manière à être ouverte par un élément intermédiaire métallique ou en polyéthylène entre la cupule et l'insert, tel qu'une bague.

On connaît différents types de prothèses cotyloïdiennes fixées par expansion, comprenant trois éléments qui sont :
- un cotyle artificiel consistant en une cupule pourvue de fentes d'expansion qui sont symétriquement et régulièrement réparties angulairement sur sa périphérie et qui déterminent des segments ou portions de secteur de calotte sphérique ;
- un élément intermédiaire fileté qui est :
   a) une bague destinée à être vissée dans le cotyle, comme décrit dans la demande de brevet EP 486 403, ou
   b) un noyau intermédiaire qui comporte un doigt fileté faisant appendice sur son pôle et qui coopère avec un trou taraudé dans le cotyle artificiel, comme décrit dans la demande de brevet FR 2 700 946 ;
- un insert de frottement qui vient s'appuyer ou s'emboîter sur ledit élément intermédiaire présentant une surface de glissement en forme de cavité hémisphérique, et dans laquelle vient s'emboîter une boule céphalique artificielle pour reconstituer l'articulation de la hanche.

Une prothèse selon le préambule de la revendication 1 est décrit dans la demande de brevet FR-A-2645433.

La présente invention concerne les prothèses cotyloïdiennes expansibles à trois éléments, comme décrit ci-dessus. Plus particulièrement, la présente invention concerne des prothèses cotyloïdiennes à trois éléments substantiellement de forme de calotte sphérique.

Avec les prothèses cotyloïdiennes expansibles actuelles, l'insert en matière plastique qui doit encaisser tous les efforts d'appui sur la hanche, peut fluer et donc se déformer à l'intérieur du cotyle.

Dans le cas d'élément intermédiaire à surface continue qui supporte mieux l'insert, le cotyle est fixé par vis, et n'assure pas non plus une bonne stabilité d'ensemble : les pièces de la prothèse conservent en effet une certaine mobilité et élasticité qui sont également incompatibles avec une bonne tenue dans le temps.

La présente invention vise à pallier à ces inconvénients en particulier, la présente invention vise à fournir une prothèse présentant :
. une tenue mécanique améliorée,
. une mise en place dans la qualité osseuse plus aisée et plus précise,
. une usure des pièces réduite,
. la possibilité de permettre une double mobilité grâce à un insert mobile dans l'élément intermédiaire.

Pour ce faire, la présente invention a pour objet une prothèse cotyloïdienne comprenant :
- un cotyle artificiel creux en forme de cupule, de préférence de surface extérieure du type calotte sphérique, notamment hémisphérique, comportant des fentes d'expansion réparties sur sa périphérie ;
- un élément intermédiaire en forme de cupule de surface intérieure en forme de calotte sphérique, notamment hémisphérique ;
- un insert creux de surfaces extérieure et intérieure en forme de calotte sphérique, de préférence hémisphérique, se logeant dans l'élément intermédiaire;
- ledit cotyle et ledit élément intermédiaire comportent des moyens de vissage tels que la surface extérieure dudit élément intermédiaire est apte à venir en contact congruent par vissage contre au moins une génératrice intérieure et méridienne de chaque segment délimité par deux desdites fentes consécutives,
- lesdits moyens de vissage dudit élément intermédiaire dans ledit cotyle comprennent un filetage périphérique réalisé sur la surface intérieure du cotyle proche de son ouverture, filetage dans lequel se visse ledit élément intermédiaire à l'aide d'un filetage complémentaire sur sa surface extérieure proche de son ouverture,
- le diamètre interne de l'ouverture dudit cotyle en position d'attente avant le vissage dudit élément intermédiaire, est inférieur au diamètre externe dudit élément intermédiaire, et
- ledit cotyle est doté de dents d'ancrage osseux sur sa face extérieure.
   Cette configuration de la prothèse cotyloïdienne selon l'invention, dans laquelle l'élément intermédiaire s'appuie en contact sur au moins une génératrice intérieure et méridienne de la cavité intérieure du cotyle artificiel, permet une bonne répartition des efforts et des contraintes sur toute la surface du cotyle artificiel.
   En outre, le filetage périphérique selon la présente invention, assure une ouverture progressive de l'élément à fentes, de sorte que le positionnement du cotyle lors de sa mise en place est rendu plus précis.
   Plus particulièrement, l'élément intermédiaire est un noyau creux de surface extérieure hémisphérique, et apte à venir en contact contre au moins une génératrice intérieure et méridienne de chaque segment défini par deux fentes d'expansion consécutives du cotyle artificiel. De préférence, ce noyau intermédiaire est une pièce pleine dont la surface extérieure est continue. Avantageusement, toutes les surfaces internes du cotyle sont en contact avec la surface extérieure du noyau, quand celui-ci est entièrement vissé et mis en place dans le cotyle.
   Pour assurer ce vissage, la partie intérieure proche de l'ouverture du cotyle creux comporte un filetage dans lequel se visse ledit noyau intermédiaire disposant également d'un filetage correspondant au niveau de son bord extérieur supérieur entourant sa propre ouverture, et le diamètre interne de l'ouverture dudit cotyle en position d'attente avant le vissage dudit noyau intermédiaire, est inférieur à celui externe de ce noyau intermédiaire.
   Le résultat est une nouvelle prothèse cotyloïdienne expansible à double mobilité, qui apporte des solutions aux problèmes posés par les inconvénients relevés sur les prothèses actuelles et tels qu'explicités précédemment : en effet, les trois éléments qui composent la prothèse suivant l'invention, sont tous de forme hémisphérique emboîtable et congruente. Ainsi, du fait du contact continu de la surface externe de la pièce intermédiaire avec celle interne du cotyle inférieur, et qui sont en parfaite congruence une fois mises en place, on obtient un meilleur appui que lorsque la pièce intermédiaire n'est qu'une bague et/ou fixée au cotyle que par l'intermédiaire de filets permettant leur vissage respectif : la présente invention évite dans le temps toute mobilité et déplacement entre ces pièces.
   De plus, la surface intérieure continue de l'élément intermédiaire permet de la même façon une congruence parfaite de l'insert en matière plastique qui ne risquera pas ainsi de fluer ou de s'accrocher dans une quelconque aspérité de l'élément intermédiaire.
   Le fait que les trois pièces constituant la prothèse selon la présente invention soient en contact et congruentes les unes par rapport aux autres, permet d'assurer un contact sans élasticité : cette conception est très différente de la plupart des brevets publiés sur ce type de prothèse et va à l'encontre même de l'enseignement relevé jusqu'à ce jour, tel que par exemple celui du brevet EP 640325, puisqu'au contraire ceux-ci en général recherchent un minimum d'élasticité entre les différentes pièces.
   La présente invention permet, grâce à cet emboîtage et la congruence de l'élément intermédiaire avec le cotyle, de régler l'orientation de l'insert qui reçoit ensuite la boule céphalique de l'articulation, comme cela est décrit dans la demande de brevet précédente de M. Chauvin, citée ci-dessus.
   Par ailleurs, les position et disposition particulières des dents d'ancrage et de pré-impaction extérieures du cotyle, selon la présente invention, permettent d'une part, de mettre en place celui-ci dans la cavité osseuse de manière aisée et précise par simple appui au fond de celle-ci sans avoir à tarauder la paroi de cette cavité, permettant ainsi de stabiliser le cotyle dans l'os pour que le vissage de l'élément intermédiaire soit possible, et d'autre part de s'ancrer dans cette cavité osseuse par expansion lors du vissage de noyau intermédiaire, l'orientation des ailettes formant lesdites dents d'ancrage assurant à la fois l'anti-rotation et l'anti-extraction dudit cotyle. On obtient une stabilité première très efficace grâce au lien ainsi obtenu entre la prothèse et l'os.
   Dans un mode de réalisation particulièrement avantageux, les dents d'ancrage sont réparties régulièrement sur la surface extérieure desdits segments du cotyle artificiel vers son ouverture et les extrémités distales desdites dents d'ancrage avant vissage sont situées à l'intérieur de la surface enveloppe, de préférence d'une sphère enveloppe de diamètre Ds, correspondant à la position de la surface externe du cotyle une fois mis en expansion par vissage de l'élément intermédiaire.
   Plus particulièrement, les dents d'ancrage disposées sur la surface extérieure des segments vers l'ouverture du cotyle sont formées d'ailettes, les unes étant symétriques par rapport à un plan méridien passant par l'axe polaire du cotyle, et les autres étant symétriques par rapport à un plan perpendiculaire audit axe.
   Selon une variante avantageuse de réalisation de l'invention, lesdits segments comportent des dents d'impaction consistant en des picots situées dans la zone polaire de la surface extérieure du cotyle, de préférence.
   La zone proche de l'ouverture du cotyle dans laquelle sont disposées les dents d'ancrage et la zone polaire dans laquelle sont disposées les dents d'impaction, sont délimitées par un plan perpendiculaire à l'axe polaire dans une proportion de 2/3 à 2/5 pour la zone proche de l'ouverture et 1/5 à 1/3 pour la zone polaire.
   Lesdits picots d'impaction sont disposés selon une direction verticale ou intermédiaire entre la direction verticale de l'axe polaire et la direction radiale de ladite sphère de diamètre et
- les dents d'ancrage osseux sont disposées selon une direction radiale ou intermédiaire entre ladite direction radiale et la direction perpendiculaire à l'axe polaire.

Dans un mode de réalisation avantageux, la surface intérieure dudit élément intermédiaire est pleine et polie, et ledit insert consiste en une cupule fixe ou mobile, de préférence mobile, de surface externe en forme de calotte sphérique lisse, et présente une cavité à surface intérieure sphérique lisse.

La possibilité d'utiliser une cupule mobile s'articulant avec la surface intérieure de l'élément intermédiaire poli, permet d'assurer une plus grande stabilité à l'articulation prothétique dans les mouvements extrêmes, ce qui diminue le risque de luxation.

Dans un autre mode de réalisation, il est possible d'utiliser un insert fixe : dans cette configuration, l'invention offre aussi l'avantage de la congruence parfaite entre l'insert et la cupule, pour réduire l'usure et le fluage.

Les caractéristiques selon lesquelles la surface intérieure de l'élément intermédiaire est lisse et/ou cette surface est pleine, c'est-à-dire parfaitement régulière non perforée, ne présentant aucune aspérité ou cavité (trou de vis), représentent un avantage important car cela évite la production de débris d'usure de l'insert, notamment lorsqu'il est en polyéthylène.

D'autres caractéristiques et avantages de la présente invention apparaîtront à la lecture de la description détaillée qui va suivre, en référence avec les figures 1 à 3.

La figure 1 est une coupe transversale représentant la prothèse cotyloïdienne suivant l'invention, introduite dans la cavité de l'os iliaque d'un patient déjà opéré.

Les figures 2a et 2b représentent respectivement en vue de côté et en coupe, un cotyle artificiel suivant l'invention.

Les figures 3a et 3b représentent respectivement en vue de côté et en coupe, un élément intermédiaire suivant l'invention.

La prothèse cotyloïdienne expansible 1 est du type de celle comportant un cotyle artificiel 2 creux dans lequel vient se visser un élément intermédiaire 3 qui reçoit un insert 4, également creux et en matière plastique tel que du polyéthylène, ou autres matériaux telle que céramique.

Le cotyle artificiel 2 et l'élément intermédiaire 3 sont de préférence réalisés dans une matière métallique biocompatible.

Le cotyle artificiel en forme de calotte creuse 2 comporte un certain nombre de fentes d'expansion 6 qui sont régulièrement et symétriquement réparties sur sa périphérie : les fentes 6 sont ménagées en direction du pôle P₂ du cotyle de manière à former entre chaque fente un segment 7, l'ensemble de ces segments étant solidaires de la calotte polaire P₂, qui est donc indéformable dans cette zone et dont le diamètre extérieur D₅ correspond au diamètre de la cavité osseuse 5, réalisée dans l'os iliaque 10, afin de recevoir ladite prothèse. Cette zone polaire P₂ est bien sûr située suivant l'axe polaire P₁ P₁' de la prothèse et à l'opposé des différentes ouvertures des différentes pièces qui la composent.

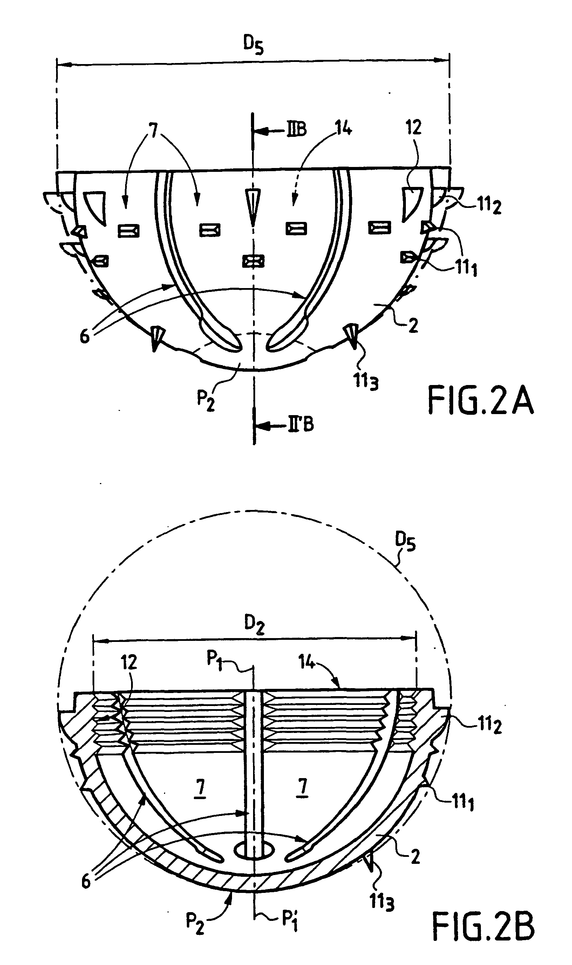

Telles que représentées sur la figure 2b, les extrémités distales des dents 11₁ et 11₂ d'ancrage, disposées vers l'ouverture 14 du cotyle artificiel 2 sont situées à l'intérieur de la sphère enveloppe de diamètre D₅ correspondant à la position de la surface externe du cotyle 2, une fois mis en expansion par l'élément intermédiaire 3 : ceci facilite l'insertion du cotyle dans la cavité osseuse 5. En revanche, les dents d'impaction 11₃ en forme de picots, situées sur les segments 7 vers la zone polaire P₂ dudit cotyle 2, dépassent de ladite sphère enveloppe de diamètre D₅ afin d'être impactées directement dans la cavité osseuse 5 lors de la mise en place dudit cotyle 2 au fond de celle-ci et d'assurer une première immobilisation.

La zone proche de l'ouverture 14 du cotyle 12 dans laquelle sont disposées les dents d'ancrage 11₁ et 11₂ et la zone polaire P₂ dans laquelle sont disposées les dents d'impaction 11₃, sont délimitées par un plan perpendiculaire à l'axe polaire P₁, P'₁ dans une proportion de 3/4 pour la zone proche de l'ouverture 14 et 1/4 pour la zone polaire, c'est-à-dire que la zone polaire occupe une hauteur le long de l'axe polaire à partir dudit pôle correspondant à 1/4 de la hauteur totale.

Sur la figure 2, ladite zone polaire P₂ du cotyle 2 sur laquelle sont disposés les picots d'impaction 11₃ du cotyle 2 dans la cavité osseuse, est une calotte sphérique et forme avec les extrémités distales des dents d'ancrage osseux 11 disposées hors de ladite zone polaire P₂ vers l'ouverture du cotyle 14 en position d'attente avant vissage, une sphère de diamètre D₅ correspondant à la position de la surface extérieure dudit cotyle 2 après vissage et ancrage des dents 11 dans la cavité osseuse, comme représenté en traits espacés sur la figure 2B.

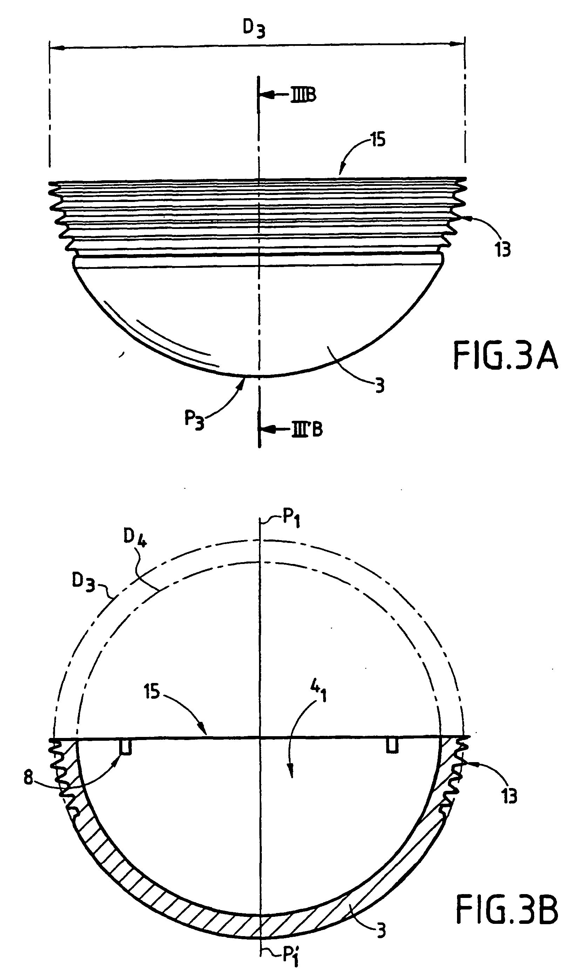

Sur la figure 3, l'élément intermédiaire 3 est d'un profil extérieur correspondant à celui interne du cotyle 2 une fois expansé, mais avec un diamètre extérieur D₃ supérieur au diamètre interne d₂ dudit cotyle au niveau de l'ouverture 14 de celui-ci quand il est en position d'attente par exemple lors de sa mise en place à l'intérieur de la cavité 5 contre le fond de celle-ci : ainsi quand on visse l'élément intermédiaire 3 à l'intérieur du cotyle 2, on écarte progressivement lesdits segments 7 au fur et à mesure que les filets du filetage 13 de l'élément intermédiaire 3, s'engagent dans ceux du filetage 12 du cotyle 2 du fait de leur disposition en profil divergent sur la surface sphérique extérieure dudit élément intermédiaire. L'écartement progressif desdits segments 7 obtenu ainsi par vissage de l'élément intermédiaire 3 dans le cotyle 2 permet une fixation souple et sans contrainte par ancrage progressif des dents 11₁ et 11₂ dans la paroi de la cavité osseuse 5 jusqu'à ce que la surface intérieure du cotyle corresponde à celle extérieure de l'élément intermédiaire 3, les deux surfaces ayant alors le même diamètre D₃ et celle externe du cotyle ayant simultanément atteint celui D₅ de la cavité osseuse 5.

Ces dents 11 d'ancrage, disposées sur la face externe des segments 7 vers l'ouverture 14 du cotyle 1, sont formées d'ailettes les unes 11₂ disposées dans des plans méridiens passant par l'axe polaire P₁ P'₁ du cotyle 2 et assurant une fonction d'anti-rotation, et les autres 11₁ dans des plans parallèles coupant perpendiculairement l'axe P₁ P'₁ et assurant la fonction d'anti-extraction.

Une fois l'élément intermédiaire 3 entièrement vissé dans le cotyle artificiel 2, leur surface respectivement extérieure D₃ et intérieure d₂ étant en contact continu, on peut introduire l'insert en plastique 4 à l'intérieur de l'élément intermédiaire 3 à travers son ouverture 15 et les surfaces respectivement interne de l'élément 3 et externe de l'insert 4 étant de même diamètre D₄, viennent également en contact l'une contre l'autre.

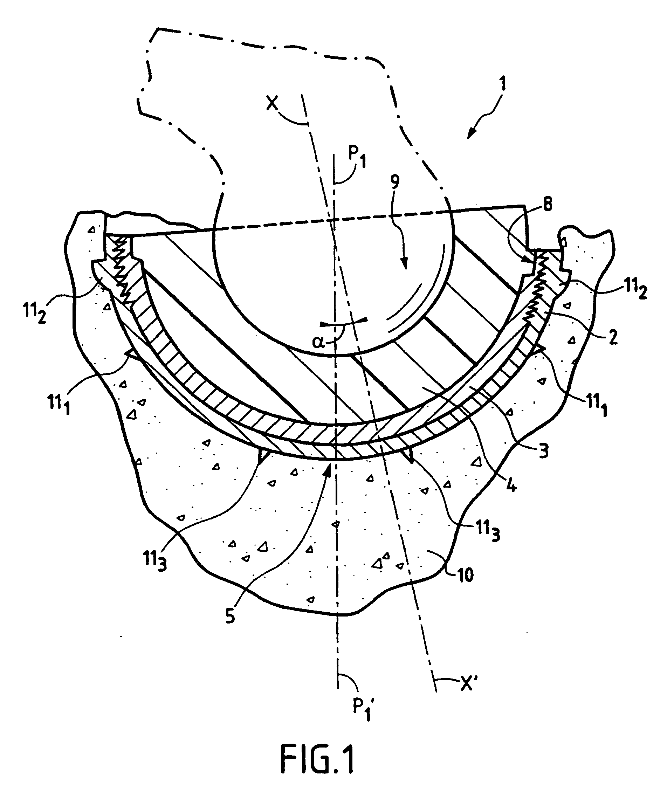

Cet insert 4 réalisé de préférence en matière plastique comporte lui-même en son milieu une cavité 9 qui permet de recevoir la tête prothétique représentée en traits mixtes sur la figure 1. Pour réaliser un insert fixe, l'insert 4 comprendra, disposés en couronne sur sa face externe, des appendices qui sont régulièrement répartis sur sa périphérie : ces appendices présentent un profil externe identique au jeu près à celui d'encoches 8 ménagées dans l'élément intermédiaire 3, autour et au bord de son ouverture 15, et dans lesquelles viennent se loger lesdits appendices de l'insert 4 : l'inclinaison de la couronne portant lesdits appendices par rapport à l'axe XX' polaire de l'insert 4 permet de régler angulairement celui-ci par rapport à l'axe polaire P₁ P'₁ de l'élément intermédiaire 3 et du cotyle 2 ; de même en ce qui concerne la position angulaire des appendices, par rapport aux encoches 8 pour régler l'angle de rotation de l'insert 4 autour de l'axe polaire P₁ P'₁ ; ceci étant décrit dans la demande de brevet FR 2 700 946 de M. Chauvin. Des moyens de fixation et de blocage dudit insert 4 par rapport à l'élément intermédiaire 3 sont également décrits dans cette demande de brevet FR 2 700.946.

Dans une variante de réalisation, la surface intérieure du cotyle 2 et la surface extérieure de l'élément intermédiaire 3, ont une forme partiellement tronconique ou de calotte non sphérique à courbure ovoïde présentant toutefois de préférence une ouverture circulaire de diamètre interne d₂.

L'insert 4 peut être constitué en matériau céramique.

La surface extérieure dudit cotyle artificiel 2 peut être traitée avec un biomatériau favorisant l'ostéointégration tel que l'hydroxyapathite (HAP).

Enfin, des pattes et/ou crochets peuvent être ajoutés et disposés dans la zone proche de l'ouverture 14 du cotyle 2.

## Revendications

1. Prothèse cotyloïdienne expansible comprenant :
- un cotyle artificiel creux (2) en forme de cupule, de préférence de surface extérieure du type calotte sphérique, comportant des fentes d'expansion (6) réparties sur sa périphérie ;
- un élément intermédiaire (3) en forme de cupule de surface intérieure en forme de calotte sphérique ;
- un insert (4) creux de surfaces extérieure et intérieure en forme de calotte sphérique, de préférence hémisphérique, se logeant dans l'élément intermédiaire (3);
- ledit cotyle (2) et ledit élément intermédiaire (3) comportent des moyens de vissage tels que la surface extérieure dudit élément intermédiaire (3) est apte à venir en contact congruent par vissage contre au moins une génératrice intérieure et méridienne de chaque segment (7) délimité par deux desdites fentes (6) consécutives;
- ledit cotyle est doté de dents (11) d'ancrage osseux sur sa face extérieure;
**caractérisée en ce que:**
- lesdits moyens de vissage dudit élément intermédiaire (3) dans ledit cotyle (2) comprennent un filetage périphérique réalisé sur la surface intérieure du cotyle (2) proche de son ouverture (12), filetage dans lequel se visse ledit élément intermédiaire (3) à l'aide d'un filetage complémentaire sur sa surface extérieure proche de son ouverture,
- le diamètre (d₂) interne de l'ouverture (14) dudit cotyle (2) en position d'attente avant le vissage dudit élément intermédiaire (3), est inférieur au diamètre (D₃) externe dudit élément intermédiaire (3).

2. Prothèse cotyloïdienne suivant la revendication 1, **caractérisée en ce que** l'élément intermédiaire (3) est une pièce pleine dont la surface extérieure est continue.

3. Prothèse cotyloïdienne suivant la revendication 2, **caractérisée en ce que** toutes les surfaces internes du cotyle (2) sont en contact avec la surface extérieure de l'élément (3) quand celui-ci est entièrement vissé et mis en place dans le cotyle (2).

4. Prothèse cotyloïdienne suivant l'une quelconque des revendications 1 à 3, **caractérisée en ce que** la surface intérieure du cotyle (2) et la surface extérieure de l'élément intermédiaire (3) sont en forme de calotte sphérique, de préférence hémisphérique.

5. Prothèse cotyloïdienne suivant l'une quelconque des revendications 1 à 3, **caractérisée en ce que** les dents (11) d'ancrage sont réparties régulièrement sur la surface extérieure desdits segments (7) du cotyle artificiel (2) vers son ouverture (12) et les extrémités distales desdites dents d'ancrage (11) avant vissage sont situées à l'intérieur de la surface enveloppe, de préférence d'une sphère enveloppe de diamètre (D₅) correspondant à la position de la surface externe du cotyle (2) une fois mis en expansion par vissage de l'élément intermédiaire (3).

6. Prothèse cotyloïdienne suivant l'une des revendications 1 à 5, **caractérisée en ce que** les dents (11) d'ancrage disposées sur la surface extérieure des segments (7) vers l'ouverture (14) du cotyle (2) sont formées d'ailettes, les unes (11₂) étant symétriques par rapport à un plan méridien passant par l'axe polaire (P₁ P'₁) du cotyle, et les autres (11₁) étant symétriques par rapport à un plan perpendiculaire audit axe (P₁ P'₁).

7. Prothèse cotyloïdienne suivant l'une quelconque des revendications 1 à 6, **caractérisée en ce que** lesdits segments (7) comportent des dents d'impaction consistant en des picots (11₃) situées dans une zone polaire (P₂) de la surface extérieure du cotyle (2).

8. Prothèse cotyloïdienne suivant l'une quelconque des revendications 1 à 7, **caractérisée en ce que** la zone proche de l'ouverture (14) du cotyle (2) dans laquelle sont disposées les dents d'ancrage (11₁ et 11₂) et la zone polaire (P₂) dans laquelle sont disposées les dents d'impaction (11₃), sont délimitées par un plan perpendiculaire à l'axe polaire (P₁, P'₁) dans une proportion de 2/3 à 2/5 pour la zone proche de l'ouverture (14) et 1/5 à 1/3 pour la zone polaire (P₂).

9. Prothèse cotyloïdienne suivant l'une quelconque des revendications 1 à 8, **caractérisée en ce que** ladite zone polaire (P₂) du cotyle (2) sur laquelle sont disposés les picots d'impaction (11₃) du cotyle (2) dans la cavité osseuse, est une calotte sphérique et forme avec les extrémités distales des dents d'ancrage osseux (11) disposées hors de ladite zone polaire (P₂) vers l'ouverture du cotyle (14) en position d'attente avant vissage, une sphère de diamètre (D₅) correspondant à la position de la surface extérieure dudit cotyle (2) après vissage et ancrage des dents (11) dans la cavité osseuse.

10. Prothèse cotyloïdienne suivant l'une quelconque des revendications 7 à 9, **caractérisée en ce que** lesdits picots d'impaction (11₃) sont disposés selon une direction verticale ou intermédiaire entre la direction verticale de l'axe polaire (P₁, P'₁) et la direction radiale de ladite sphère de diamètre (D₅) et
- les dents d'ancrage osseux (11₁ et 11₂) sont disposées selon une direction radiale ou intermédiaire entre ladite direction radiale et la direction perpendiculaire à l'axe polaire (P₁, P'₁).

11. Prothèse cotyloïdienne suivant l'une quelconque des revendications 1 à 10, **caractérisée en ce que** la surface intérieure dudit élément intermédiaire (3) est pleine et polie, et ledit insert (4) consiste en une cupule de préférence mobile de surface externe en forme de calotte sphérique lisse, et présente une cavité à surface intérieure sphérique lisse.

12. Prothèse cotyloïdienne suivant l'une quelconque des revendications 1 à 11, **caractérisée en ce que** ledit insert (4) est constitué en matériau plastique ou céramique.

13. Prothèse cotyloïdienne suivant l'une quelconque des revendications 1 à 12, **caractérisée en ce que** ledit cotyle artificiel (2) et ledit élément intermédiaire (3) sont réalisés en métal biocompatible.

14. Prothèse selon l'une des revendications 1 à 13, **caractérisée en ce que** le cotyle artificiel (2) est recouvert avec un biomatériau favorisant l'ostéointégration.

15. Prothèse selon l'une des revendications 1 à 14, **caractérisée en ce que** des pattes ou des crochets sont ajoutés dans la zone proche de l'ouverture (14) du cotyle (2).

## Patentansprüche

1. Dehnbare Gelenkpfannenprothese, umfassend:
- eine hohle künstliche Gelenkpfanne (2) in Form eines Bechers, vorzugsweise mit einer äußeren Oberfläche des Typs einer kugelförmigen Kuppel, die über ihren Umfang verteilt Dehnungsspalten (6) trägt;
- ein Zwischenelement (3) in Form eines Bechers mit einer inneren Oberfläche in Form einer kugelförmigen Kuppel;
- einen hohlen Einsatz (4) mit einer äußeren und inneren Oberfläche in Form einer kugelförmigen, vorzugsweise halbkugelförmigen Kuppel, der sich in dem Zwischenelement (3) befindet, wobei die Gelenkpfanne (2) und das Zwischenelement (3) derartige Schraubvorrichtungen tragen, dass die äußere Oberfläche des Zwischenelementes (3) in der Lage ist, mit mindestens einer inneren und Meridian-Mantellinie jedes Segmentes (7), das von zwei aufeinander folgenden Spalten (6) begrenzt wird, durch Einschrauben kongruent in Kontakt zu treten, wobei die Gelenkpfanne an ihrer Außenfläche mit Knochenverankerungszähnen (11) ausgestattet ist;
**dadurch gekennzeichnet, dass**
- die Schraubvorrichtungen des Zwischenelementes (3) in der Gelenkpfannen (2) ein peripheres Gewinde umfassen, das an der inneren Oberfläche der Gelenkpfanne (2) in der Nähe ihrer Öffnung (12) hergestellt worden ist, wobei in das Gewinde das Zwischenelement (3) mithilfe eines komplementären Gewindes auf seiner äußeren Oberfläche in der Nähe seiner Öffnung eingeschraubt wird,
- der Innendurchmesser (d₂) der Öffnung (14) der Gelenkpfanne (2) in Bereitschaftsstellung vor dem Einschrauben des Zwischenelementes (3) kleiner als der Außendurchmesser (D₃) des Zwischenelementes (3) ist.

2. Gelenkpfannenprothese nach Anspruch 1, **dadurch gekennzeichnet, dass** das Zwischenelement (3) ein massives Teil mit durchgehender äußerer Oberfläche ist.

3. Gelenkpfannenprothese nach Anspruch 2, **dadurch gekennzeichnet, dass** alle inneren Oberflächen der Gelenkpfanne (2) mit der äußeren Oberfläche des Elementes (3) in Kontakt stehen, wenn dieses vollständig eingeschraubt und in der Gelenkpfanne (2) an Ort und Stelle gebracht ist.

4. Gelenkpfannenprothese nach einem der Ansprüche 1 bis 3, **dadurch gekennzeichnet, dass** die innere Oberfläche der Gelenkpfanne (2) und die äußere Oberfläche des Zwischenelementes (3) die Form einer kugelförmigen, vorzugsweise halbkugelförmigen, Kuppel haben.

5. Gelenkpfannenprothese nach einem der Ansprüche 1 bis 3, **dadurch gekennzeichnet, dass** die Verankerungszähne (11) auf der äußeren Oberfläche der Segmente (7) der künstlichen Gelenkpfanne (2) in Richtung ihrer Öffnung (12) gleichmäßig verteilt sind und sich die distalen Enden der Verankerungszähne (11) vor dem Einschrauben im Inneren der Mantelfläche, vorzugsweise einer Kugelfläche mit dem Durchmesser (D₅), befinden, die der Position der äußeren Oberfläche der Gelenkpfanne, die durch Einschrauben des Zwischenelementes (3) gedehnt wurde, entspricht.

6. Gelenkpfannenprothese nach einem der Ansprüche 1 bis 5, **dadurch gekennzeichnet, dass** die auf der äußeren Oberfläche der Segmente (7) in Richtung der Öffnung (14) der Gelenkpfanne (2) angeordneten Verankerungszähne (11) aus Rippen gebildet werden, von denen die einen (11₂) in Bezug auf eine Meridianebene, die durch die Polarachse (P₁, P'₁) der Gelenkpfanne geht, symmetrisch sind und die anderen (11₁) in Bezug auf eine Ebene, die senkrecht zu dieser Achse (P₁ P'₁) verläuft, symmetrisch sind.

7. Gelenkpfannenprothese nach einem der Ansprüche 1 bis 6, **dadurch gekennzeichnet, dass** die Segmente (7) Einschlagzähne tragen, die aus Spitzen (11₃) bestehen, die in einer Polarzone (P₂) der äußeren Oberfläche der Gelenkpfanne (2) angeordnet sind.

8. Gelenkpfannenprothese nach einem der Ansprüche 1 bis 7, **dadurch gekennzeichnet, dass** die Zone in der Nähe der Öffnung (14) der Gelenkpfanne (2), in der die Verankerungszähne (11₁ und 11₂) angeordnet sind, und die Polarzone (P₂), in der die Einschlagzähne (11₃) angeordnet sind, durch eine Ebene, die senkrecht zur Polarachse (P₁, P₁') verläuft, in einem Verhältnis von 2/3 bis 2/5 für die Zone in der Nähe der Öffnung (14) und 1/5 bis 1/3 für die Polarzone (P₂) begrenzt werden.

9. Gelenkpfannenprothese nach einem der Ansprüche 1 bis 8, **dadurch gekennzeichnet, dass** die Polarzone (P₂) der Gelenkpfanne (2), auf der die Einschlagspitzen (11₃) der Gelenkpfanne (2) angeordnet sind, in der Knochenhöhlung eine kugelförmige Kuppel ist und mit den distalen Enden der Knochenverankerungszähne (11), die sich außerhalb der Polarzone (P₂) in Richtung der Öffnung der Gelenkpfanne (14) befinden, in Bereitschaftsstellung vor dem Einschrauben eine Kugel mit dem Durchmesser (D₅) bilden, die der Position der äußeren Oberfläche der Gelenkpfanne (2) nach Einschrauben und Verankern der Zähne (11) in der Knochenhöhlung entspricht.

10. Gelenkpfannenprothese nach einem der Ansprüche 7 bis 9, **dadurch gekennzeichnet, dass** die Einschlagspitzen (11₃) in vertikaler oder intermediärer Richtung zwischen der vertikalen Richtung der Polarachse (P₁, P'₁) und der radialen Richtung der Kugel mit dem Durchmesser (D₅) angeordnet sind und
- die Knochenverankerungszähne (11₁ und 11₂) in radialer oder intermediärer Richtung zwischen dieser radialen Richtung und der zur Polachse (P₁, P'₁) senkrechten Richtung angeordnet sind.

11. Gelenkpfannenprothese nach einem der Ansprüche 1 bis 10, **dadurch gekennzeichnet, dass** die innere Oberfläche des Zwischenelementes (3) massiv und poliert ist und der Einsatz (4) aus einem vorzugsweise beweglichen Becher mit einer äußeren Oberfläche in Form einer kugelförmigen glatten Kuppel besteht und eine Höhlung mit einer kugelförmigen glatten inneren Oberfläche aufweist.

12. Gelenkpfannenprothese nach einem der Ansprüche 1 bis 11, **dadurch gekennzeichnet, dass** der Einsatz (4) aus einem Kunststoff- oder Keramikmaterial besteht.

13. Gelenkpfannenprothese nach einem der Ansprüche 1 bis 12, **dadurch gekennzeichnet, dass** die künstliche Gelenkpfanne (2) und das Zwischenelement (3) aus einem biokompatiblen Metall hergestellt sind.

14. Prothese nach einem der Ansprüche 1 bis 13, **dadurch gekennzeichnet, dass** die künstliche Gelenkpfanne (2) mit einem Biomaterial beschichtet ist, das die Osteointegration fördert.

15. Prothese nach einem der Ansprüche 1 bis 14, **dadurch gekennzeichnet, dass** Klammern oder Haken in der Zone in der Nähe der Öffnung (14) der Gelenkpfanne (2) hinzugefügt sind.

## Claims

1. Expansible acetabular prosthesis comprising:
- a hollow artificial acetabular cup (2), preferably with an outer surface of the spherical cap type, having expansion slots (6) distributed over its periphery;
- an intermediate cup-shaped element (3), with an inner surface of spherical cap shape,
- a hollow insert (4) with outer and inner surfaces of spherical cap shape, preferably hemispherical, fitting in the intermediate element (3);
- said acetabular cup is equipped with bone-anchoring teeth (11) on its outer face;
- said acetabular cup (2) and said intermediate element (3) comprise screwing means so that the outer surface of said intermediate element (3) can come into congruent contact, by screwing, against at least one internal meridian generatrix of each segment (7) delimited by two of said consecutive slots (6);
**characterized in that**
- said means of screwing said intermediate element (3) into said acetabular cup (2) comprise a peripheral threading formed on the inner surface of the acetabular cup (2) near its opening (12), in which threading said intermediate element (3) is screwed with the aid of a complementary threading on its outer surface near its opening,
- the internal diameter (d₂) of the opening (14) of said acetabular cup (2), in the standby position before screwing of said intermediate element (3), is smaller than the external diameter (D₃) of said intermediate element (3).

2. Acetabular prosthesis according to Claim 1, **characterized in that** the intermediate element (3) is a solid component whose outer surface is continuous.

3. Acetabular prosthesis according to Claim 2, **characterized in that** all the inner surfaces of the acetabular cup (2) are in contact with the outer surface of the element (3) when the latter is fully screwed and positioned in the acetabular cup (2).

4. Acetabular prosthesis according to any one of Claims 1 to 3, **characterized in that** the inner surface of the acetabular cup (2) and the outer surface of the intermediate element (3) are of spherical cap shape, preferably hemispherical.

5. Acetabular prosthesis according to any one of Claims 1 to 3, **characterized in that** the anchoring teeth (11) are distributed uniformly over the outer surface of said segments (7) of the artificial acetabular cup (2) toward its opening (12), and, before screwing, the distal ends of said anchoring teeth (11) are situated inside the enveloping surface, preferably an enveloping sphere of diameter (D₅) corresponding to the position of the outer surface of the acetabular cup (2) once expanded by screwing of the intermediate element (3).

6. Acetabular prosthesis according to one of Claims 1 to 5, **characterized in that** the anchoring teeth (11) arranged on the outer surface of the segments (7) toward the opening (14) of the acetabular cup (2) are formed as fins, some (11₂) being symmetrical with respect to a meridian plane passing through the polar axis (P₁, P'₁) of the acetabular cup, and the others (11₁) being symmetrical with respect to a plane perpendicular to said axis (P₁, P'₁).

7. Acetabular prosthesis according to any one of Claims 1 to 6, **characterized in that** said segments (7) comprise impaction teeth consisting of spikes (11₃) situated in a polar zone (P₂) of the outer surface of the acetabular cup (2).

8. Acetabular prosthesis according to any one of Claims 1 to 7, **characterized in that** the zone near the opening (14) of the acetabular cup (2), in which the anchoring teeth (11₁ and 11₂) are arranged, and the polar zone (P₂), in which the impaction teeth (11₃) are arranged, are delimited by a plane perpendicular to the polar axis (P₁, P'₁) in a proportion of 2/3 to 2/5 for the zone near the opening (14) and of 1/5 to 1/3 for the polar zone (P₂).

9. Acetabular prosthesis according to any one of Claims 1 to 8, **characterized in that** said polar zone (P₂) of the acetabular cup (2), on which the impaction spikes (11₃) of the acetabular cup (2) are arranged in the bone cavity, is a spherical cap and forms, with the distal ends of the bone-anchoring teeth (11) arranged outside said polar zone (P₂) toward the opening of the acetabular cup (14), in the standby position before screwing, a sphere of diameter (D₅) corresponding to the position of the outer surface of said acetabular cup (2) after screwing and anchoring of the teeth (11) in the bone cavity.

10. Acetabular prosthesis according to any one of Claims 7 to 9, **characterized in that** said impaction spikes (11₃) are arranged in a vertical direction or an intermediate direction between the vertical direction of the polar axis (P₁, P'₁) and the radial direction of said sphere of diameter (D₅) and
- the bone-anchoring teeth (11₁ and 11₂) are arranged in a radial direction or an intermediate direction between said radial direction and the direction perpendicular to the polar axis (P₁, P'₁).

11. Acetabular prosthesis according to any one of Claims 1 to 10, **characterized in that** the inner surface of said intermediate element (3) is solid and polished, and said insert (4) consists of a cup, preferably movable, whose outer surface is the shape of a smooth spherical cap and which has a cavity with a smooth spherical inner surface.

12. Acetabular prosthesis according to any one of Claims 1 to 11, **characterized in that** said insert (4) is made of plastic or ceramic material.

13. Acetabular prosthesis according to any one of Claims 1 to 12, **characterized in that** said artificial acetabular cup (2) and said intermediate element (3) are made of biocompatible metal.

14. Prosthesis according to one of Claims 1 to 13, **characterized in that** the artificial acetabular cup (2) is covered with a biomaterial which promotes osseointegration.

15. Prosthesis according to one of Claims 1 to 14, **characterized in that** lugs or hooks are added in the zone near the opening (14) of the acetabular cup (2).
